# EUROPEAN PATENT SPECIFICATION

(11) **EP 3 266 831 B1**
(45) Date of publication and mention of the grant of the patent: **05.05.2021**
(21) Application number: 16758642.9
(22) Date of filing: 03.03.2016
(51) Int. Cl.: C08L 67/04, C08J 3/22, C08K 3/34, C08K 5/053

(54) **METHOD OF PRODUCING POLYESTER RESIN COMPOSITION AND METHOD OF PRODUCING POLYESTER RESIN FORMED ARTICLE, AND POLYESTER RESIN COMPOSITION AND POLYESTER RESIN FORMED ARTICLE**
VERFAHREN ZUR HERSTELLUNG EINER POLYESTERHARZZUSAMMENSETZUNG UND VERFAHREN ZUR HERSTELLUNG EINES AUS POLYESTERHARZ GEFORMTEN GEGENSTANDES SOWIE POLYESTERHARZZUSAMMENSETZUNG UND AUS POLYESTERHARZ GEFORMTER GEGENSTAND
PROCÉDÉ DE PRODUCTION D'UNE COMPOSITION DE RÉSINE DE POLYESTER ET PROCÉDÉ DE PRODUCTION D'UN ARTICLE FORMÉ EN RÉSINE DE POLYESTER, ET COMPOSITION DE RÉSINE DE POLYESTER ET ARTICLE FORMÉ EN RÉSINE DE POLYESTER

(30) Priority: 05.03.2015 JP 2015044022
(43) Date of publication of application: 10.01.2018
(73) Proprietor: Kaneka Corporation, Osaka-shi, Osaka 530-8288 (JP)
(72) Inventor: MINAMI, Tetsuya, Osaka 566-0072 (JP); SUZUKI, Noriyuki, Osaka 566-0072 (JP)
(74) Representative: Vossius & Partner Patentanwälte Rechtsanwälte mbB
(86) International application number: PCT/JP2016/001154
(87) International publication number: WO 2016/139946

(56) References cited:
- EP-A1- 3 056 541
- EP-A1- 3 246 361
- WO-A1-2004/101683
- WO-A1-2013/116763
- JP-A- 2006 525 136
- JP-A- 2010 142 986
- JP-A- 2010 143 978
- JP-A- 2014 136 773
- US-A1- 2015 353 707
- A. EL-HADI ET AL.: "Correlation between degree of crystallinity, morphology,glass temperature, mechanical properties and biodegradationof poly (3-hydroxyalkanoate) PHAs and their blends", POLYMER TESTING, vol. 21, 1 February 2002 (2002-02-01), pages 665-674, XP055510352,

## Description

### Technical Field

The present invention relates to a method of producing a polyester resin composition and a method of producing a polyester resin formed article, and to the polyester resin composition and the polyester resin formed article.

### Background Art

Large amounts of petroleum-based plastics are disposed of every year, and serious problems caused by the large amounts of wastes, such as shortage of landfills and environment pollution, have been discussed. Under the circumstances, biodegradable plastics have been drawing attention since biodegradable plastics are degraded by the action of microorganisms in environments such as soil or seawater, in landfills, or in composts. Biodegradable plastics are under development with the aim of expanding their application to materials for use in the aforementioned environments in agriculture, forestry, and fisheries, and also to food containers, packaging materials, sanitary materials, garbage bags, etc., which are difficult to recover/recycle after use.

In consideration of the aforementioned serious problems and from the viewpoint of carbon dioxide emission reduction or carbon dioxide fixation (carbon neutral), polyhydroxyalkanoates (hereinafter, abbreviated as "PHA" in some cases), which are plant-derived aliphatic polyesters, have been drawing attention. Among polyhydroxyalkanoates, in particular, polylactic acids (hereinafter, abbreviated as "PLA" in some cases) have been drawing attention because lactic acid, which is the raw material of the polylactic acids, is inexpensive for the reason that it is produced by fermentation using sugars that are extracted from corn, potato, or the like, and also because polylactic acid resins are highly rigid and highly transparent.

However, the glass-transition temperature of such a polylactic acid is around 55°C. Therefore, there is a problem, for example, that the thermal resistance of the polylactic acid is insufficient and the application thereof is limited. In addition, since the crystallization speed of the polylactic acid is slow, even if the polylactic acid is kept around 100°C at which its crystallization is most accelerated, it takes long for the polylactic acid to be crystallized completely. Thus, there is also a problem of poor productivity. In order to improve the thermal resistance and processability, there is proposed a method of mixing a polylactic acid resin with another resin and a soluble azo lake pigment serving as a crystal nucleating agent (Patent Literature 1).

Also, there is disclosed an aliphatic polyester resin composition that contains a polyhydroxyalkanoate and pentaerythritol for the purpose of improving the processability of the aliphatic polyester resin and suppressing burr formation on the resin (Patent Literature 2).

### Citation List

### Patent Literature

PTL 1: Japanese Laid-Open Patent Application Publication No. 2010-132816
PTL 2: WO 2014/020838

### Summary of Invention

### Technical Problem

However, currently, substantially high advantages are not obtained in terms of thermal resistance, and further improvements are necessary. In view of the current circumstances, an object of the present invention is to provide a method of producing a polyester resin composition and a method of producing a polyester resin formed article that make it possible to improve the thermal resistance, which is a drawback of polylactic acids, and to provide the polyester resin composition and the polyester resin formed article.

### Solution to Problem

The inventors of the present invention conducted diligent studies to improve the thermal resistance of a polylactic acid. As a result of the studies, they have found that the thermal resistance of a polylactic acid is significantly improved by producing a resin composition containing four components that are a polylactic acid, a poly(3-hydroxyalkanoate), pentaerythritol, and a silicate by a particular production method. Based on this finding, they have arrived at the present invention.

Specifically, the present invention provides: a method of producing a polyester resin composition as described below in [1] to [6]; a polyester resin composition as described below in [7]; a method of producing a polyester resin formed article as described below in [8] to [10]; and a polyester resin formed article as described below in [11].
[1] A method of producing a polyester resin composition, the method including: a step (I-a) of obtaining a polylactic acid composition (X) containing a polylactic acid (A), pentaerythritol (C), and a silicate (D); and a step (II-a) of mixing the polylactic acid composition (X) with a poly(3-hydroxyalkanoate) (B).
[2] A method of producing a polyester resin composition, the method including: a step (I-b) of obtaining a poly(3-hydroxyalkanoate) composition (Y) containing a poly(3-hydroxyalkanoate) (B), pentaerythritol (C), and a silicate (D); and a step (II-b) of mixing the poly(3-hydroxyalkanoate) composition (Y) with a polylactic acid (A).
[3] The method of producing a polyester resin composition according to the above [1] or [2], in which the pentaerythritol (C) is blended such that an amount of the pentaerythritol (C) is not less than 0.05 parts by weight and not more than 20 parts by weight with respect to 100 parts by weight of a total amount (A + B) of the polylactic acid (A) and the poly(3-hydroxyalkanoate) (B).
[4] The method of producing a polyester resin composition according to any one of the above [1] to [3], in which the silicate (D) is blended such that an amount of the silicate (D) is not less than 10 parts by weight and not more than 40 parts by weight with respect to 100 parts by weight of a total amount (A + B) of the polylactic acid (A) and the poly(3-hydroxyalkanoate) (B).
[5] The method of producing a polyester resin composition according to any one of the above [1] to [4], in which the polylactic acid (A) is blended such that an amount of the polylactic acid (A) is not less than 55 parts by weight and not more than 75 parts by weight with respect to 100 parts by weight of a total amount (A + B) of the polylactic acid (A) and the poly(3-hydroxyalkanoate) (B).
[6] The method of producing a polyester resin composition according to any one of the above [1] to [5], in which the poly(3-hydroxyalkanoate) (B) is at least one selected from the group consisting of poly(3-hydroxybutyrate), poly(3-hydroxybutyrate-co-3-hydroxyvalerate), poly(3-hydroxybutyrate-co-3-hydroxyvalerate-co-3-hydroxyhexanoate), poly(3-hydroxybutyrate-co-3-hydroxyhexanoate), and poly(3-hydroxybutyrate-co-4-hydroxybutyrate).
[7] A polyester resin composition manufactured by the method of producing a polyester resin composition according to any one of the above [1] to [6].
[8] A method of producing a polyester resin formed article, the method including: after the step (II-a) according to the above [1], subjecting the polyester resin composition to forming.
[9] A method of producing a polyester resin formed article, the method including: after the step (II-b) according to the above [2], subjecting the polyester resin composition to forming.
[10] The method of producing a polyester resin formed article according to the above [8] or [9], in which a temperature of a mold during the forming is 25 to 55°C.
[11] A polyester resin formed article produced by the method of producing a polyester resin formed article according to any one of the above [8] to [10].

### Advantageous Effects of Invention

The present invention makes it possible to improve thermal resistance, which is a drawback of polylactic acids.

### Description of Embodiments

Hereinafter, embodiments of a method of producing a polyester resin composition, a method of producing a polyester resin formed article, the polyester resin composition, and the polyester resin formed article according to the present invention are described in detail. However, the present invention is not limited to the embodiments described below.

### [Polyester Resin Composition]

The polyester resin composition produced by the production method of the present invention contains a polylactic acid (A), a poly(3-hydroxyalkanoate) (B), pentaerythritol (C), and a silicate (D). Hereinafter, the polylactic acid (A), the poly(3-hydroxyalkanoate) (B), the pentaerythritol (C), and the silicate (D) are described.

### [Polylactic Acid (A)]

The polylactic acid (A) used in the present invention contains a repeating unit represented by a general formula (1) shown below.
[Chem. 1]

[-CH(CH₃)-CO-O-] General Formula (1)

Preferably, the polylactic acid (A) is at least one selected from polylactic acids each containing the repeating unit represented by the general formula (1).

The polylactic acid (A) used in the present invention preferably contains 50 mol% or more of the repeating unit represented by the general formula (1) with respect to the total repeating units, and may contain other repeating units.

The polylactic acid (A) may be poly(D-lactic acid), poly(L-lactic acid), a copolymer of D-lactic acid and L-lactic acid, or a stereocomplex formed by blending these.

If forming (i.e., processing) is performable, there is no particular restriction in terms of molecular weight and molecular weight distribution. However, it is preferable to use a polylactic acid whose weight-average molecular weight is 50,000 to 300,000 from the viewpoint of excellent balance between physical properties and processability of the obtained formed article. More preferably, the weight-average molecular weight of the polylactic acid is 100,000 to 250,000.

It should be noted that the weight-average molecular weight of the polylactic acid is obtained from a molecular weight distribution (in terms of polystyrene) that is measured by using gel permeation chromatography (GPC) using a chloroform solution. A column used in the GPC may be any column suitable for measuring the molecular weight. In the present invention, a commercially available polylactic acid, for example, "Ingeo" (registered trademark) available from NatureWorks LLC, "REVODE" (registered trademark) available from Zhejiang Hisun Biomaterials Co., Ltd., or one available from Corbion, can be used.

The amount of the polylactic acid used in the present invention is such that the lower limit amount of the polylactic acid (A) is preferably not less than 55 parts by weight, more preferably not less than 58 parts by weight, and even more preferably not less than 60 parts by weight with respect to 100 parts by weight of the total amount (A + B) of the polylactic acid (A) and the poly(3-hydroxyalkanoate) (B) from the viewpoint of excellent burr formation suppressing effect of the obtained formed article. The upper limit amount of the polylactic acid is preferably not more than 75 parts by weight, more preferably not more than 73 parts by weight, and even more preferably not more than 70 parts by weight with respect to 100 parts by weight of the total amount (A + B) of the polylactic acid and the poly(3-hydroxyalkanoate) from the viewpoint of excellent thermal resistance.

### [Poly(3-Hydroxyalkanoate) (B)]

The poly(3-hydroxyalkanoate) (B) used in the present invention contains a repeating unit represented by a general formula (2) shown below.
[Chem. 2]

[-CHR-CH₂-CO-O-] General Formula (2)

(In the general formula (2), R is an alkyl group represented by CₙH₂ₙ₊₁ and n is an integer of not less than 1 and not more than 15.)

Preferably, the poly(3-hydroxyalkanoate) (B) is at least one selected from microorganism-derived poly(3-hydroxyalkanoates) (hereinafter, poly(3-hydroxyalkanoate) is abbreviated as "P3HA" in some cases) that are produced from microorganisms and each of which contains the repeating unit represented by the general formula (2).

The poly(3-hydroxyalkanoate) (B) used in the present invention preferably contains 50 mol% or more of the repeating unit represented by the general formula (2) with respect to the total repeating units, and may contain other repeating units.

The microorganisms that produce the P3HAs are not particularly limited, so long as they have the ability to produce P3HAs. For example, Bacillus megaterium is the first discovered poly(3-hydroxybutyrate)-producing microorganism (hereinafter, poly(3-hydroxybutyrate) is abbreviated as "P3HB" in some cases), which was discovered in 1925, and natural microorganisms such as Cupriavidus necator (formerly classified as Alcaligenes eutrophus, Ralstonia eutropha) and Alcaligenes latus are known as other P3HB-producing microorganisms. These microorganisms accumulate P3HB in their cells.

Further, known microorganisms that produce copolymers of hydroxybutyrate and another hydroxyalkanoate are, for example, Aeromonas caviae, which produces poly(3-hydroxybutyrate-co-3-hydroxyvalerate) (hereinafter, abbreviated as "P3HB3HV" in some cases) and poly(3-hydroxybutyrate-co-3-hydroxyhexanoate) (hereinafter, abbreviated as "P3HB3HH" in some cases), and Alcaligenes eutrophus, which produces poly(3-hydroxybutyrate-co-4-hydroxybutyrate) (hereinafter, abbreviated as "P3HB4HB" in some cases). In particular, a more preferred P3HB3HH-producing microorganism is, for example, Alcaligenes eutrophus AC32, FERM BP-6038 (T. Fukui, Y. Doi, J. Bateriol., 179, p. 4821-4830 (1997)), in which a P3HA synthase gene is introduced to improve P3HB3HH productivity. These microorganisms are cultured under proper conditions, and the resulting microorganism cells having P3HB3HH accumulated therein are used. Other than the above microorganisms, genetically-modified microorganisms may also be used, in which various P3HA synthesis-related genes are introduced in accordance with the intended type of P3HA to be produced, and culture conditions including the type of a substrate may be optimized.

The molecular weight of the P3HA used in the present invention is not particularly limited, so long as the P3HA substantially exhibits sufficient physical properties for the intended use. However, if the molecular weight is too low, the obtained formed article has low strength. On the other hand, if the molecular weight is too high, the processability is reduced, and therefore preparing the formed article is difficult. In consideration of these, the weight-average molecular weight of the P3HA used in the present invention is preferably in the range of 200,000 to 2,500,000, more preferably in the range of 250,000 to 2,000,000, and even more preferably in the range of 300,000 to 1,000,000. It should be noted that the weight-average molecular weight of the P3HA is obtained from a molecular weight distribution (in terms of polystyrene) that is measured by using gel permeation chromatography (GPC) using a chloroform solution. A column used in the GPC may be any column suitable for measuring the molecular weight.

The P3HA used in the present invention is preferably a polymer resin that contains 80 mol% or more of 3-hydroxybutyrate. More preferably, the P3HA used in the present invention is a polymer resin that contains 85 mol% or more of 3-hydroxybutyrate and that is produced by a microorganism. Specific examples of the P3HA used in the present invention include poly(3-hydroxybutyrate), poly(3-hydroxybutyrate-co-3-hydroxypropionate),
poly(3-hydroxybutyrate-co-3-hydroxyvalerate),
poly(3-hydroxybutyrate-co-3-hydroxyvalerate-3-hydroxyhexanoate),
poly(3-hydroxybutyrate-co-3-hydroxyhexanoate),
poly(3-hydroxybutyrate-co-3-hydroxyheptanoate),
poly(3-hydroxybutyrate-co-3-hydroxyoctanoate),
poly(3-hydroxybutyrate-co-3-hydroxynonanoate),
poly(3-hydroxybutyrate-co-3-hydroxydecanoate),
poly(3-hydroxybutyrate-co-3-hydroxyundecanoate), and
poly(3-hydroxybutyrate-co-4-hydroxybutyrate). In particular, from the viewpoint of processability or physical properties of the formed article, poly(3-hydroxybutyrate),
poly(3-hydroxybutyrate-co-3-hydroxyvalerate),
poly(3-hydroxybutyrate-co-3-hydroxyvalerate-3-hydroxyhexanoate),
poly(3-hydroxybutyrate-co-3-hydroxyhexanoate), and
poly(3-hydroxybutyrate-co-4-hydroxybutyrate) are suitably usable.

From the viewpoint of, for example, processability, productivity, or the quality of the formed article, the composition ratio of 3-hydroxybutyrate (hereinafter, abbreviated as "3HB" in some cases) to a comonomer copolymerizable therewith (e.g., 3-hydroxyvalerate (hereinafter, abbreviated as "3HV" in some cases), 3-hydroxyhexanoate (hereinafter, abbreviated as "3HH" in some cases), or 4-hydroxybutyrate (hereinafter, abbreviated as "4HB" in some cases)) in the poly(3-hydroxyalkanoate) (B) used in the present invention, that is, the ratio of monomers in the poly(3-hydroxyalkanoate) (B) copolymer resin, is preferably 3-hydroxybutyrate/comonomer = 97/3 to 80/20 (mol%/mol%), and more preferably 95/5 to 85/15 (mol%/mol%).

The ratio of each monomer in the poly(3-hydroxyalkanoate) (B) can be measured by gas chromatography in a manner described below. In a vessel, 2 ml of a sulfuric acid/methanol mixed liquid (15/85 (weight ratio)) and 2 ml of chloroform are added to about 20 mg of the dry poly(3-hydroxyalkanoate) (B), and the vessel is tightly sealed. Then, the resulting mixture is heated at 100°C for 140 minutes to obtain a methyl ester as a poly(3-hydroxyalkanoate) (B) degradation product. After cooling the mixture, 1.5 g of sodium hydrogen carbonate is added thereto little by little for neutralization, and the resulting mixture is left as it is until generation of carbon dioxide stops. The mixture is well mixed with 4 ml of diisopropyl ether added thereto, and then the monomer unit composition of the poly(3-hydroxyalkanoate) (B) degradation product in a supernatant is analyzed by capillary gas chromatography to determine the ratio of each monomer in the poly(3-hydroxyalkanoate) (B).

The gas chromatography is performed by using "GC-17A" manufactured by Shimadzu Corporation as a gas chromatograph and "NEUTRA BOND-1" (with a column length of 25 m, a column inner diameter of 0.25 mm, and a liquid film thickness of 0.4 µm) manufactured by GL Sciences Inc. as a capillary column. He gas is used as a carrier gas; the column inlet pressure is set to 100 kPa; and a sample is injected in an amount of 1 µl. Temperature conditions are as follows: the temperature is increased from an initial temperature of 100°C to 200°C at a rate of 8 °C/min, and is further increased from 200°C to 290°C at a rate of 30 °C/min.

The blending amount of the poly(3-hydroxyalkanoate) (B) used in the present invention is such that the lower limit blending amount of the poly(3-hydroxyalkanoate) (B) is preferably not less than 25 parts by weight, more preferably not less than 28 parts by weight, and even more preferably not less than 30 parts by weight with respect to 100 parts by weight of the total amount (A + B) of the polylactic acid (A) and the poly(3-hydroxyalkanoate) (B) from the viewpoint of excellent thermal resistance. The upper limit blending amount of the P3HA (B) is preferably not more than 45 parts by weight, more preferably not more than 42 parts by weight, and even more preferably not more than 40 parts by weight with respect to 100 parts by weight of the total amount (A + B) of the polylactic acid (A) and the poly(3-hydroxyalkanoate) (B) from the viewpoint of excellent burr formation suppressing effect of the obtained formed article.

### [Pentaerythritol (C)]

The pentaerythritol (C) used in the present invention is a polyhydric alcohol represented by a general formula (3) shown below.

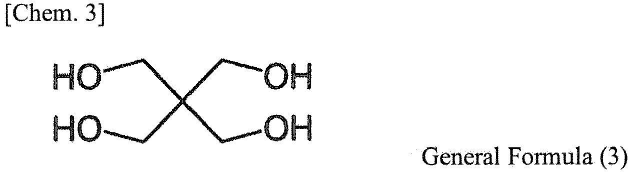

The pentaerythritol (C) is one type of polyhydric alcohol represented by the general formula (3), and is a white crystalline organic compound having a melting point of 260.5°C. The pentaerythritol (C) is classified as a sugar alcohol that is not derived from a natural product and that can be synthesized by condensation of acetaldehyde and formaldehyde under basic conditions.

The pentaerythritol (C) used in the present invention is not particularly limited, so long as it is commonly available, and may be one provided as a reagent or an industrial product. Examples of the reagent include, but are not limited to, those available from Wako Pure Chemical Industries, Ltd., Sigma-Aldrich, Tokyo Chemical Industries Co., Ltd., and Merck. Examples of the industrial product include, but are not limited to, one available from Koei Chemical Co., Ltd., (trade name: Pentarit), one available from The Nippon Synthetic Chemical Industry Co., Ltd. (trade name: Neulizer P), one available from Toyo Chemicals Co., Ltd., and one available from Perstorp.

Some of such commonly-available reagents and industrial products contain, as an impurity, an oligomer produced by dehydration condensation of the pentaerythritol (C), such as dipentaerythritol or tripentaerythritol. Although the oligomer does not have the effect of crystallizing polyhydroxyalkanoates, the oligomer does not inhibit the crystallization effect of the pentaerythritol (C). Therefore, the oligomer may be contained in the reagent or industrial product used in the present invention.

The blending amount of the pentaerythritol (C) used in the present invention is such that the lower limit amount of the pentaerythritol is preferably not less than 0.05 parts by weight, more preferably not less than 0.1 parts by weight, and even more preferably not less than 0.5 parts by weight with respect to 100 parts by weight of the total amount (A + B) of the polylactic acid (A) and the poly(3-hydroxyalkanoate) (B) so that the pentaerythritol (C) will exert its effect as a crystal nucleating agent to a high degree. The upper limit amount of the pentaerythritol is preferably not more than 20 parts by weight, more preferably not more than 12 parts by weight, even more preferably not more than 10 parts by weight, and most preferably not more than 8 parts by weight with respect to 100 parts by weight of the total amount (A + B) of the polylactic acid (A) and the poly(3-hydroxyalkanoate) (B) from the viewpoint of excellent flow properties of the resin during processing.

### [Silicate (D)]

The silicate (D) used in the present invention is not particularly limited, so long as it has the effect of, for example, improving the thermal resistance. For example, talc, mica, kaolinite, montmorillonite, and smectite are suitably usable since they are highly versatile, have a high effect of improving mechanical strength, have a narrow particle diameter distribution, and hardly inhibit surface smoothness and mold transferability.

Not only one type of the silicate (D) but two types thereof may be mixed. The mixture ratio is suitably adjustable in accordance with the type of the polylactic acid (A), the type of the poly(3-hydroxyalkanoate) (B), and intended effects.

The amount of the silicate (D) used in the present invention is such that the lower limit amount of the silicate is preferably not less than 10 parts by weight, more preferably not less than 13 parts by weight, and even more preferably not less than 15 parts by weight with respect to 100 parts by weight of the total amount (A + B) of the polylactic acid (A) and the poly(3-hydroxyalkanoate) (B) from the viewpoint of excellent processability. The upper limit amount of the silicate is preferably not more than 40 parts by weight, more preferably not more than 37 parts by weight, and even more preferably not more than 35 parts by weight with respect to 100 parts by weight of the total amount (A + B) of the polylactic acid (A) and the poly(3-hydroxyalkanoate) (B) from the viewpoint of excellent thermal resistance.

The silicate (D) used in the present invention may be surface-treated by adhering a surface treatment agent to the surface of the particles of the silicate (D) in order to improve its dispersibility in the polyester resin composition. Examples of the surface treatment agent include a higher fatty acid, silane coupling agent, titanate coupling agent, sol-gel coating agent, and a resin coating agent.

The water content in the silicate (D) used in the present invention is preferably 0.01 to 10%, more preferably 0.01 to 5%, and even more preferably 0.01 to 1% so that hydrolysis of the polylactic acid (A) and the poly(3-hydroxyalkanoate) (B) can be readily suppressed. It should be noted that the water content is measured by a method complying with JIS-K5101.

The mean particle diameter of the silicate (D) used in the present invention is preferably 0.1 to 100 µm, and more preferably 0.1 to 50 µm, from the viewpoint of excellent polyester resin composition properties and processability. It should be noted that the mean particle diameter is measured by a method using a laser diffraction/scattering device, such as "Microtrac MT3100 II" manufactured by Nikkiso Co., Ltd.

It should be noted that both the silicate (D) used in the present invention and the pentaerythritol (C) have a function as a crystal nucleating agent. By allowing the silicate (D) to coexist with the pentaerythritol (C), the crystallization of the polyester resin composition can be further accelerated and the processability thereof can be improved.

Examples of the silicate (D) used in the present invention are indicated below.

In the case of using talc as the silicate (D), examples thereof include general-purpose talc and surface-treated talc. Specific examples of the talc used as the silicate (D) include "MICRO ACE" (registered trademark) available from Nippon Talc Co., Ltd., "Talcan powder" (registered trademark) available from Hayashi-Kasei Co., Ltd., and talc products available from TAKEHARA KAGAKU KOGYO CO., LTD. and MARUO CALCIUM CO., LTD.

In the case of using mica as the silicate (D), examples thereof include wet ground mica and dry ground mica. Specific examples of the mica used as the silicate (D) include mica products available from YAMAGUCHI MICA CO., LTD. and Keiwa Rozai Co., Ltd.

In the case of using kaolinite as the silicate (D), examples thereof include dry kaolin, baked kaolin, and wet kaolin. Specific examples of the kaolinite used as the silicate (D) include "TRANSLINK" (registered trademark), "ASP" (registered trademark), "SANTINTONE" (registered trademark), and "ULTREX" (registered trademark) available from Hayashi-Kasei Co., Ltd. and a kaolinite product available from Keiwa Rozai Co., Ltd.

### [First Polyester Resin Composition Production Method]

A first polyester resin composition production method according to the present invention includes a step (I-a) and a step (II-a) described below. The step (I-a) is the step of obtaining a polylactic acid composition (X) containing the polylactic acid (A), the pentaerythritol (C), and the silicate (D). The step (II-a) is the step of mixing the polylactic acid composition (X) with the poly(3-hydroxyalkanoate) (B).

### [Step (I-a)]

Through this step, the polylactic acid composition (X) containing the polylactic acid (A), the pentaerythritol (C), and the silicate (D) can be obtained. The polylactic acid composition (X) (hereinafter, referred to as a "master batch" in some cases) is a polyester resin composition in which at least one of the pentaerythritol (C) and the silicate (D) is contained at a higher concentration than in the polyester resin composition obtained by the production method of the present invention.

One specific example of the step (I-a) is as follows. The polylactic acid (A), the pentaerythritol (C), the silicate (D), and other components (if necessary) are added into, and melt-kneaded by, an extruder, kneader, Banbury mixer, rolls, or the like to prepare a resin composition, which is extruded as a strand and then cut to obtain pellets of the polylactic acid composition (X). The particle shape of the pellets is, for example, columnar, elliptical columnar, spherical, cubic, or rectangular parallelepiped.

For the above melt-kneading of the polylactic acid (A), the pentaerythritol (C), the silicate (D), and others, the melt-kneading temperature cannot be uniformly specified since the melt-kneading temperature depends on, for example, the melting point and melt viscosity of the polylactic acid in use. However, for example, the temperature of the melt-kneaded resin at the die hole is preferably 160 to 200°C, more preferably 165 to 195°C, and even more preferably 170 to 190°C from the viewpoint of suppressing thermal degradation or obtaining high kneadability.

The polylactic acid (A), the pentaerythritol (C), and the silicate (D) may be added into the kneader at the same time for melt-kneading. Alternatively, the polylactic acid (A) may be melted first, and then the pentaerythritol (C) and the silicate (D) may be added thereto.

It is preferable to add the silicate (D) at last from the viewpoint of not deteriorating the properties of the obtained polyester resin composition or formed article. That is, it is preferable to add the silicate (D) to a resin composition that has been obtained by melt-kneading the polylactic acid (A) and the pentaerythritol (C) at an intended ratio. Among various examples of the silicate (D), in general, talc and mica contain water and indicate alkalinity. Therefore, if such talc or mica coexists with a polyhydroxyalkanoate such as the polylactic acid (A) at high temperature for a long period of time, degradation of the polyhydroxyalkanoate may be accelerated, and thereby the mechanical properties of the resin composition may deteriorate.

Specifically, for example, in the case of preparing a resin composition by an intermeshed co-rotation twin screw extruder, it is preferable that the polylactic acid (A) and the pentaerythritol (C) be added from the root of the screws (i.e., from the main hopper), and that the silicate (D) be added by, for example, side feeding at the downstream side of the extruder.

### [Step (II-a)]

In this step, the polylactic acid composition (X) prepared in the step (I-a) is mixed with the poly(3-hydroxyalkanoate) (B) and other components if necessary. Preferably, before mixing the polylactic acid composition (X) prepared in the step (I-a) with the poly(3-hydroxyalkanoate) (B), the polylactic acid composition (X) prepared in the step (I-a) is sufficiently dried at 40 to 80°C to remove water therefrom.

### [Second Polyester Resin Composition Production Method]

A second polyester resin composition production method according to the present invention includes the steps (I-a) and (II-a), but in the second polyester resin composition production method, the polylactic acid (A) in the step (I-a) and the poly(3-hydroxyalkanoate) (B) in the step (II-a) are switched with each other. The second polyester resin composition production method can be performed in the same manner as the above-described polyester resin composition production method.

In other words, the second polyester resin composition production method according to the present invention includes a step (I-b) and a step (II-b) described below. The step (I-b) is the step of obtaining a poly(3-hydroxyalkanoate) composition (Y) (hereinafter, referred to as a "master batch" in some cases) containing the poly(3-hydroxyalkanoate) (B), the pentaerythritol (C), and the silicate (D). The step (II-b) is the step of mixing the poly(3-hydroxyalkanoate) composition (Y) with the polylactic acid (A).

Also in the case where the polylactic acid (A) in the step (I-a) and the poly(3-hydroxyalkanoate) (B) in the step (II-a) are switched with each other, the thermal resistance, which is a drawback of the polylactic acid, can be improved similar to the above-described polyester resin composition production method including the step (I-a) and the step (II-a) (i.e., the first polyester resin composition production method).

The polyester resin composition produced by each production method of the present invention has excellent processability, which cannot be realized by conventional resin compositions containing a polyhydroxyalkanoate resin. The excellent processability of the polyester resin composition allows the resin temperature during melting in processing and the temperature and cooling time of a resin cooling medium, such as a mold, to be set over wide ranges.

The polyester resin composition according to the present invention contains the polylactic acid (A), the poly(3-hydroxyalkanoate) (B), the pentaerythritol (C), and the silicate (D), and in addition, the polyester resin composition may contain other components, such as an antioxidant, an ultraviolet absorber, a colorant such as a dye or pigment, a plasticizer, a lubricant, an inorganic filler, an organic filler, or an antistatic agent. The additive amounts of these other components are not particularly limited, so long as advantageous effects of the present invention are not impaired.

The polyester resin composition according to the present invention is excellent in terms of thermal resistance, and it can be processed in a short time and is suitable for use as a base material of, for example, tableware, agricultural materials, parts for OA equipment, parts for home appliances, automobile components, everyday sundries, stationery, various molded bottles, extruded sheets, or profile-extruded products.

### [Polyester Resin Formed Article]

After the step (II-a) or the step (II-b) of the polyester resin composition production method, a polyester resin formed article can be produced by subjecting the polyester resin composition to forming.

One example of the case of subjecting the polyester resin composition to forming after the step (II-a) is as follows. In the step (II-a), the poly(3-hydroxyalkanoate) (B) and other components (if necessary) can be added to the polylactic acid composition (X) obtained in the step (I-a), which can be then directly subjected to processing by a known processing method, and thereby an intended formed article can be obtained (hereinafter, a forming method in which two or more types of different materials in the shape of, for example, pellets are mixed together without melting them and the resulting mixture is directly subjected to forming is referred to as dry-blend forming in some cases). Also in the case of subjecting the polyester resin composition to forming after the step (II-b), the processing can be performed by the same processing method, and thereby an intended formed article can be obtained.

Regarding temperatures during forming, the temperature of the resin is preferably 160 to 200°C and more preferably 170 to 190°C from the viewpoint of excellent appearance in terms of, for example, coloring. Also, the temperature of the resin cooling medium, such as a mold, is preferably 25 to 55°C and more preferably 30 to 50°C during forming.

Examples of an adoptable processing method include injection molding, extrusion forming, film forming, sheet forming, blow molding, fiber spinning, extrusion foaming, or expanded bead molding.

Examples of an adoptable injection molding method include: an injection molding method commonly used to mold a thermoplastic resin; a gas assist molding method; and an injection compression molding method. Also, according to intended use, any injection molding method other than the above methods is adoptable, such as an in-mold forming method, a gas press molding method, a two-color molding method, a sandwich molding method, PUSH-PULL, or SCORIM. It should be noted that adoptable injection molding methods are not limited to these examples.

Examples of an adoptable film forming method include T-die extrusion forming, calendaring, roll forming, and film blowing. It should be noted that adoptable film forming methods are not limited to these examples.

In a case where the polyester resin composition or formed article of the present invention is a film, the film can be subjected to thermoforming by heating, vacuum forming, or press molding.

In the case of subjecting the poly(3-hydroxyalkanoate) (B) to processing by injection molding, the molten resin enters a gap at joining portions (e.g., parting line portions, inserting portions, or slide-core sliding portions) of a cavity in a shaping mold for injection molding, and form "burrs" which adhere to the formed article. Since the poly(3-hydroxyalkanoate) (B) is slow in terms of crystallization and the resin keeps its fluidity for a long period of time, the burrs are easily formed and after-treatment of the formed article requires a lot of labor. However, the polyester resin composition according to the present invention, which contains the polylactic acid (A), the poly(3-hydroxyalkanoate) (B), the pentaerythritol (C), and the silicate (D), is solidified fast. For this reason, the burr formation hardly occurs. This makes is possible to reduce the labor for the after-treatment of the formed article. Therefore, the polyester resin composition according to the present invention is preferable in terms of practicality.

### Examples

Hereinafter, the present invention is specifically described with reference to Examples, but the technical scope of the present invention is not limited by these Examples.

Polylactic acid (A): products indicated below were used.
PLA-1: Ingeo 3251D manufactured by NatureWorks LLC
PLA-2: Ingeo 3260HP manufactured by NatureWorks LLC

Poly(3-hydroxyalkanoate) (B): those obtained in Production Examples and products indicated below were used.
P3HA-1: one obtained in P3HA Production Example 1 was used.

### <P3HA Production Example 1>

Culture production was performed by using KNK-005 strain (see U.S. Patent No. 7384766). The composition of a seed medium was: 1 w/v% Meat-extract, 1 w/v% Bacto-Tryptone, 0.2 w/v% Yeast-extract, 0.9 w/v% Na₂HPO₄•12H₂O, and 0.15 w/v% KH₂PO₄ (pH 6.8). The composition of a preculture medium was: 1.1 w/v% Na₂HPO₄•12H₂O, 0.19 w/v% KH₂PO₄, 1.29 w/v% (NH₄)₂SO₄, 0.1 w/v% MgSO₄•7H₂O, and 0.5 v/v% trace metal salt solution (prepared by dissolving, in 0.1 N hydrochloric acid, 1.6 w/v% FeCl₃•6H₂O, 1 w/v% CaCl₂•2H₂O, 0.02 w/v% COCl₂•6H₂O, 0.016 w/v% CuSO₄•5H₂O, and 0.012 w/v% NiCl₂•6H₂O). Palm oil was added at one time as a carbon source at a concentration of 10 g/L.

The composition of a PHA production medium was: 0.385 w/v% Na₂HPO₄•12H₂O, 0.067 w/v% KH₂PO₄, 0.291 w/v% (NH₄)₂SO₄, 0.1 w/v% MgSO₄•7H₂O, 0.5 v/v% trace metal salt solution (prepared by dissolving, in 0.1 N hydrochloric acid, 1.6 w/v% FeCl₃•6H₂O, 1 w/v% CaCl₂•2H₂O, 0.02 w/v% CoCl₂•6H₂O, 0.016 w/v% CuSO₄•5H₂O, and 0.012 w/v% NiCl₂•6H₂O), and 0.05 w/v% BIOSPUREX 200K (defoaming agent: manufactured by Cognis Japan Ltd.).

First, a glycerol stock (50 µl) of KNK-005 strain was inoculated into the seed medium (10 ml) and seed-cultured for 24 hours. Then, the resulting seed culture was inoculated at 1.0 v/v% into a 3-liter jar fermenter (MDL-300 manufactured by B. E. MARUBISHI Co., Ltd.) containing 1.8 L of the preculture medium. Preculture was performed for 28 hours under operation conditions where a culture temperature was 33°C, a stirring speed was 500 rpm, and a ventilation volume was 1.8 L/min while pH was controlled to be in the range of 6.7 to 6.8. The pH control was performed by using a 14% aqueous ammonium hydroxide solution.

Then, the resulting preculture was inoculated at 1.0 v/v% into a 10-liter jar fermenter (MDS-1000 manufactured by B. E. MARUBISHI Co., Ltd.) containing 6 L of the production medium. Culture was performed under operation conditions where a culture temperature was 28°C, a stirring speed was 400 rpm, and a ventilation volume was 6.0 L/min while pH was controlled to be in the range of 6.7 to 6.8. The pH control was performed by using a 14% aqueous ammonium hydroxide solution. Palm oil was used as a carbon source. The culture was performed for 64 hours. After the completion of the culture, cells were collected by centrifugal separation, washed with methanol, and lyophilized, and the weight of the dried cells was measured.

One hundred milliliters of chloroform was added to one gram of the obtained dried cells, and the resulting mixture was stirred at room temperature all day and night to extract the P3HA from the cells. The mixture was filtered to remove the cell residue, and then the resulting filtrate was concentrated by an evaporator until its total volume became 30 ml. Thereafter, 90 ml of hexane was gradually added to the filtrate, and the resulting mixture was left for 1 hour in the state of being gently stirred. The mixture was filtered to separate the deposited P3HA, which was then vacuum-dried at 50°C for 3 hours. In this manner, the P3HA was obtained.

The 3HH content of the obtained P3HA was measured by gas chromatography in the following manner. In a vessel, 2 ml of a sulfuric acid-methanol mixed liquid (15:85) and 2 ml of chloroform were added to 20 mg of the dried P3HA, and the vessel was tightly sealed. Then, the resulting mixture was heated at 100°C for 140 minutes to obtain a methyl ester of P3HA degradation product. After cooling the mixture, 1.5 g of sodium hydrogen carbonate was added thereto little by little for neutralization, and the resulting mixture was left as it was until generation of carbon dioxide stopped. The mixture was well mixed with 4 ml of diisopropyl ether added thereto, and then centrifuged. Thereafter, the monomer unit composition of the polyester degradation product in a supernatant was analyzed by capillary gas chromatography.

The gas chromatography was performed by using GC-17A manufactured by Shimadzu Corporation as a gas chromatograph and NEUTRA BOND-1 (with a column length of 25 m, a column inner diameter of 0.25 mm, and a liquid film thickness of 0.4 µm) manufactured by GL Sciences Inc. as a capillary column. He gas was used as a carrier gas; a column inlet pressure was set to 100 kPa; and a sample was injected in an amount of 1 µl. Temperature conditions were as follows: the temperature was increased from an initial temperature of 100°C to 200°C at a rate of 8 °C/min, and was further increased from 200°C to 290°C at a rate of 30 °C/min.

As a result of the analysis performed under the above conditions, the PHA was found to be poly(3-hydroxybutyrate-co-3-hydroxyhexanoate) (P3HB3HH) as represented by the chemical formula (1). The content of the 3-hydroxyhexanoate (3HH) was 5.6 mol%.

After the completion of the culture, P3HB3HH was obtained from the culture solution by the method described in International Publication No. WO 2010/067543. The P3HB3HH had a weight-average molecular weight of 600,000 as measured by GPC.

P3HA-2: one obtained in P3HA Production Example 2 was used.

### <P3HA Production Example 2>

A polyhydroxyalkanoate, P3HB3HH, was obtained in the same manner as in P3HA Production Example 1, except that KNK-631 strain was used and palm kernel oil was used as a carbon source. The P3HB3HH had a weight-average molecular weight of 650,000, and the 3HH content therein was 11.4 mol%.

P3HA-3: one obtained in P3HA Production Example 3 was used.

### <P3HA Production Example 3>

By using C. necator H16 strain (ATCC17699) as a production strain, P3HB having a weight-average molecular weight of 850,000 was prepared in accordance with International Publication No. WO 09/145164. P3HA-4: EM5400F
(poly(3-hydroxybutyrate-co-4-hydroxybutyrate)) manufactured by Ecomann was used.

Other materials used in Examples and Comparative Examples are indicated below. Pentaerythritol (C): Neulizer P manufactured by The Nippon Synthetic Chemical Industry Co., Ltd.

### Silicate (D): talc (MICRO ACE K-1 manufactured by Nippon Talc Co., Ltd.)

Other polyester resin: polybutylene adipate terephthalate copolymer (hereinafter, abbreviated as "PBAT" in some cases) (Ecoflex C1200 manufactured by BASF)

Master batch: those obtained in Production Examples indicated below were used. MB-1: one obtained in Master Batch Production Example 1(hereinafter, the term "master batch" is abbreviated as "MB" in some cases) was used.

### <MB Production Example 1>

P3HA-1 as the poly(3-hydroxyalkanoate) (B), the pentaerythritol (C), and the silicate (D) were blended at a blending ratio shown in Table 1 (blending ratios shown in Table 1 presented below are expressed in part(s) by weight) and melt-kneaded by using an intermeshed co-rotation twin screw extruder (TEM-26SS manufactured by TOSHIBA MACHINE CO., LTD.) at a setting temperature of 140 to 170°C and a screw rotation speed of 100 rpm to obtain a resin composition. At the time, the resin temperature was 175°C. The temperature of the molten resin discharged from a die was directly measured with a type K thermocouple and defined as the resin temperature. The resin composition was extruded through the die to be a strand, and the strand was cut to obtain pellets of MB-1. The obtained pellets were dehumidified and dried at 80°C to remove water therefrom.

MB-2 to MB-6: those obtained in MB Production Examples 2 to 6 were used.

### <MB Production Examples 2 to 6>

Pellets of MB-2 to MB-6 were produced by the same method as that of Example 1 at blending ratios shown in Table 1.

### <Example 1>

### (Production of Polyester Resin Composition Formed Article)

Pellets of MB-1 and pellets of PLA-1 as the polylactic acid (A) were weighed at a mixture ratio shown in Table 1, and subjected to dry-blend molding. Specifically, the pellets of MB-1 and the pellets of PLA-1 were mixed together in the same plastic bag, fed into the hopper of an injection molding machine (PLASTAR Si-100IV manufactured by TOYO MACHINERY & METAL CO., LTD.), and thereby molded into a bar-shaped test piece complying with ASTM D-648 standard under the following conditions: the cylinder setting temperature of the injection molding machine was 160 to 195°C; and the setting temperature of a mold was 40°C. The composition of each formed (i.e., injection-molded) article in Table 1 is the composition of a finally obtained formed article, and is calculated from a blending ratio in MB production and a mixture ratio in dry-blend molding.

### (Deflection Temperature Under Load)

The bar-shaped test piece obtained by the injection molding was stored for one month in the atmosphere at a temperature of 23°C and a humidity of 50%. Thereafter, the test piece was subjected to deflection temperature under load measurement in accordance with the method B of ASTM D-648, and thereby the deflection temperature under load (hereinafter, abbreviated as "DTUL" in some cases) of the test piece was measured. The higher the deflection temperature under load is, the better it is. The measurement result is shown in Table 1.

### <Examples 2 to 5>

In each of Examples 2 to 5, a bar-shaped test piece of a polyester resin composition was prepared by using MB at a mixture ratio shown in Table 1 by the same method as that of Example 1, and the deflection temperature under load of the test piece was evaluated. The evaluation result of each test piece is shown in Table 1.

### <Comparative Example 1>

PLA-1 as the polylactic acid (A), P3HA-1 as the poly(3-hydroxyalkanoate) (B), the pentaerythritol (C), and the silicate (D) were compounded together at a mixture ratio shown in Table 1 by using an intermeshed co-rotation twin screw extruder under the following conditions: the setting temperature was 160 to 180°C; and the screw rotation speed was 100 rpm. As a result, a resin composition was obtained. At the time, the resin temperature was 188°C. The resulting pellets were dehumidified and dried at 80°C to remove water therefrom.

Thereafter, the dried pellets were fed into the hopper of an injection molding machine, and subjected to normal molding under the following conditions: the cylinder setting temperature of the injection molding machine was 160 to 195°C; the setting temperature of a mold was 40°C. In this manner, a bar-shaped test piece complying with ASTM D-648 standard was molded. Then, the bar-shaped test piece obtained by the injection molding was stored for one month in the atmosphere at a temperature of 23°C and a humidity of 50%. Thereafter, the test piece was subjected to deflection temperature under load measurement in accordance with the method B of ASTM D-648, and thereby the deflection temperature under load of the test piece was measured. The measurement result is shown in Table 1.

### <Comparative Examples 2 to 5>

In each of Comparative Examples 2 to 5, pellets and a bar-shaped test piece of a polyester resin composition were prepared at a mixture ratio shown in Table 1 by the same method as that of Comparative Example 1, and the deflection temperature under load of the test piece was measured. The measurement result of each test piece is shown in Table 1.

### <Comparative Example 6>

Pellets and a bar-shaped test piece of a polyester resin composition were prepared at a mixture ratio shown in Table 1 by the same method as that of Comparative Example 1. In Comparative Example 6, PBAT was used instead of the poly(3-hydroxyalkanoate) (B). The deflection temperature under load of the test piece was measured, and the measurement result is shown in Table 1.

### <Comparative Example 7>

A bar-shaped test piece of a polyester resin composition was prepared by dry-blend molding at a mixture ratio shown in Table 1 by the same method as that of Example 1. In Comparative Example 7, MB-6 using PBAT was used instead of the poly(3-hydroxyalkanoate) (B). The deflection temperature under load of the test piece was evaluated, and the evaluation result is shown in Table 1.

**[Table 1]**

| Components and Properties of Injection-Molded Article | | | | | | | (Components are expressed in units of parts by weight.) | | | | | | |
|---|---|---|---|---|---|---|---|---|---|---|---|---|---|
| | | EX. 1 | EX. 2 | EX. 3 | EX. 4 | EX. 5 | Comp. EX. 1 | Comp. EX. 2 | Comp. EX. 3 | Comp. EX. 4 | Comp. EX. 5 | Comp. EX. 6 | Comp. EX. 7 |
| Type of MB | Master Batch | MB-1 | MB-2 | MB-3 | MB-4 | MB-5 | - | - | - | - | - | - | MB-6 |
| Blending Ratio in MB Production | PLA (A) | | | | | 100 | | | | | | | |
| | P3HA (B) | 100 | 100 | 100 | 100 | | | | | | | | |
| | PBAT | | | | | | | | | | | | 100 |
| | Pentaerythritol (C) | 2.5 | 3.8 | 3.3 | 3.8 | 2.5 | | | | | | | 3.3 |
| | Silicate (D) | 75 | 75 | 67 | 75 | 50 | | | | | | | 67 |
| Mixture Ratio | MB | 54 | 54 | 42 | 54 | 76 | | | | | | | 42 |
| | PLA (A) | 46 | 46 | 58 | 46 | | 60 | 60 | 70 | 60 | 60 | 70 | 58 |
| | P3HA (B) | | | | | 24 | 40 | 40 | 30 | 40 | 40 | | |
| | PBAT | | | | | | | | | | | 30 | |
| | Pentaerythritol (C) | | | | | | 1 | 1.5 | 1 | 1.5 | 1 | 1 | |
| | Silicate (D) | | | | | | 30 | 30 | 20 | 30 | 20 | 20 | |
| Molding | Molding Method | Dry Blend | Dry Blend | Dry Blend | Dry Blend | Dry Blend | Normal | Normal | Normal | Normal | Normal | Normal | Dry Blend |
| Composition of Formed (Injection-Molded) Article | PLA-1 | 60 | | | | 60 | 60 | | | | 60 | | |
| | PLA-2 | | 60 | 70 | 60 | | | 60 | 70 | 60 | | 70 | 70 |
| | P3HA-1 | 40 | | | | 40 | 40 | | | | 40 | | |
| | P3HA-2 | | 40 | | | | | 40 | | | | | |
| | P3HA-3 | | | 30 | | | | | 30 | | | | |
| | P3HA-4 | | | | 40 | | | | | 40 | | | |
| | PBAT | | | | | | | | | | | 30 | 30 |
| | Pentaerythritol | 1 | 1.5 | 1 | 1.5 | 1 | 1 | 1.5 | 1 | 1.5 | 1 | 1 | I |
| | Silicate | 30 | 30 | 20 | 30 | 20 | 30 | 30 | 20 | 30 | 20 | 20 | 20 |
| Evaluation | DTUL (°C) | 94 | 103 | 97 | 94 | 92 | 87 | 91 | 90 | 89 | 85 | 58 | 58 |

As shown in Table 1, in Comparative Examples 1 to 5, the pellets to be used in the injection molding were compounded beforehand at one time, and then subjected to normal molding. For this reason, Comparative Examples 1 to 5 exhibited lower thermal resistance than that of Examples 1 to 5, in which dry-blend molding was adopted. In Comparative Examples 6 and 7, PBAT was used instead of the poly(3-hydroxyalkanoate) (B). For this reason, Comparative Examples 6 and 7 exhibited poor thermal resistance in both the case of adopting normal molding and the case of adopting dry-blend molding.

## Claims

1. A method of producing a polyester resin composition, the method comprising:
a step (I-a) of obtaining a polylactic acid composition (X) containing a polylactic acid (A), pentaerythritol (C), and a silicate (D); and
a step (II-a) of mixing the polylactic acid composition (X) with a poly(3-hydroxyalkanoate) (B).

2. A method of producing a polyester resin composition, the method comprising:
a step (I-b) of obtaining a poly(3-hydroxyalkanoate) composition (Y) containing a poly(3-hydroxyalkanoate) (B), pentaerythritol (C), and a silicate (D); and
a step (II-b) of mixing the poly(3-hydroxyalkanoate) composition (Y) with a polylactic acid (A).

3. The method of producing a polyester resin composition according to claim 1 or 2, wherein
the pentaerythritol (C) is blended such that an amount of the pentaerythritol (C) is not less than 0.05 parts by weight and not more than 20 parts by weight with respect to 100 parts by weight of a total amount (A + B) of the polylactic acid (A) and the poly(3-hydroxyalkanoate) (B).

4. The method of producing a polyester resin composition according to any one of claims 1 to 3, wherein
the silicate (D) is blended such that an amount of the silicate (D) is not less than 10 parts by weight and not more than 40 parts by weight with respect to 100 parts by weight of a total amount (A + B) of the polylactic acid (A) and the poly(3-hydroxyalkanoate) (B).

5. The method of producing a polyester resin composition according to any one of claims 1 to 4, wherein
the polylactic acid (A) is blended such that an amount of the polylactic acid (A) is not less than 55 parts by weight and not more than 75 parts by weight with respect to 100 parts by weight of a total amount (A + B) of the polylactic acid (A) and the poly(3-hydroxyalkanoate) (B).

6. The method of producing a polyester resin composition according to any one of claims 1 to 5, wherein
the poly(3-hydroxyalkanoate) (B) is at least one selected from the group consisting of poly(3-hydroxybutyrate), poly(3-hydroxybutyrate-co-3-hydroxyvalerate),
poly(3-hydroxybutyrate-co-3-hydroxyvalerate-co-3-hydroxyhexanoate),
poly(3-hydroxybutyrate-co-3-hydroxyhexanoate), and
poly(3-hydroxybutyrate-co-4-hydroxybutyrate).

7. A polyester resin composition manufactured by the method of producing a polyester resin composition according to any one of claims 1 to 6.

8. A method of producing a polyester resin formed article, the method comprising:
after the step (II-a) according to claim 1, subjecting the polyester resin composition to forming.

9. A method of producing a polyester resin formed article, the method comprising:
after the step (II-b) according to claim 2, subjecting the polyester resin composition to forming.

10. The method of producing a polyester resin formed article according to claim 8 or 9, wherein
a temperature of a mold during the forming is 25 to 55°C.

11. A polyester resin formed article produced by the method of producing a polyester resin formed article according to any one of claims 8 to 10.

## Patentansprüche

1. Ein Verfahren zur Herstellung einer Polyesterharzzusammensetzung, wobei das Verfahren umfasst:
einen Schritt (I-a) des Erhaltens einer Polymilchsäurezusammensetzung (X), enthaltend eine Polymilchsäure (A), Pentaerythritol (C) und ein Silikat (D); und
einen Schritt (II-a) des Mischens der Polymilchsäurezusammensetzung (X) mit einem Poly(3-hydroxyalkanoat) (B).

2. Ein Verfahren zur Herstellung einer Polyesterharzzusammensetzung, wobei das Verfahren umfasst:
einen Schritt (I-b) des Erhaltens einer Poly(3-hydroxyalkanoat)-Zusammensetzung (Y), enthaltend ein Poly(3-hydroxyalkanoat) (B), Pentaerythritol (C) und ein Silikat (D); und
einen Schritt (II-b) des Mischens der Poly(3-hydroxyalkanoat)-Zusammensetzung (Y) mit einer Polymilchsäure (A).

3. Das Verfahren zur Herstellung einer Polyesterharzzusammensetzung gemäß Anspruch 1 oder 2, wobei
das Pentaerythritol (C) derart vermischt wird, dass eine Menge des Pentaerythritols (C) nicht weniger als 0,05 Gewichtsteile und nicht mehr als 20 Gewichtsteile, bezogen auf 100 Gewichtsteile einer Gesamtmenge (A + B) der Polymilchsäure (A) und des Poly(3-hydroxyalkanoat)s (B), beträgt.

4. Das Verfahren zur Herstellung einer Polyesterharzzusammensetzung gemäß einem der Ansprüche 1 bis 3, wobei
das Silikat (D) derart vermischt wird, dass eine Menge des Silikats (D) nicht weniger als 10 Gewichtsteile und nicht mehr als 40 Gewichtsteile, bezogen auf 100 Gewichtsteile einer Gesamtmenge (A + B) der Polymilchsäure (A) und des Poly(3-hydroxyalkanoat)s (B), beträgt.

5. Das Verfahren zur Herstellung einer Polyesterharzzusammensetzung gemäß einem der Ansprüche 1 bis 4, wobei
die Polymilchsäure (A) derart vermischt wird, dass eine Menge der Polymilchsäure (A) nicht weniger als 55 Gewichtsteile und nicht mehr als 75 Gewichtsteile, bezogen auf 100 Gewichtsteile einer Gesamtmenge (A + B) der Polymilchsäure (A) und des Poly(3-hydroxyalkanoat)s (B), beträgt.

6. Das Verfahren zur Herstellung einer Polyesterharzzusammensetzung gemäß einem der Ansprüche 1 bis 5, wobei
das Poly(3-hydroxyalkanoat) (B) mindestens eines ist, ausgewählt aus der Gruppe bestehend aus Poly(3-hydroxybutyrat), Poly(3-hydroxybutyrat-co-3-hydroxyvalerat), Poly(3-hydroxybutyrat-co-3-hydroxyvalerat-co-3-hydroxyhexanoat), Poly(3-hydroxybutyrat-co-3-hydroxyhexanoat) und Poly(3-hydroxybutyrat-co-4-hydroxybutyrat).

7. Eine Polyesterharzzusammensetzung, hergestellt durch das Verfahren zur Herstellung einer Polyesterharzzusammensetzung gemäß einem der Ansprüche 1 bis 6.

8. Ein Verfahren zur Herstellung eines aus Polyesterharz geformten Gegenstandes, wobei das Verfahren umfasst:
nach dem Schritt (II-a) gemäß Anspruch 1, Unterziehen der Polyesterharzzusammensetzung einer Formgebung.

9. Ein Verfahren zur Herstellung eines aus Polyesterharz geformten Gegenstandes, wobei das Verfahren umfasst:
nach dem Schritt (II-b) gemäß Anspruch 2, Unterziehen der Polyesterharzzusammensetzung einer Formgebung.

10. Das Verfahren zur Herstellung eines aus Polyesterharz geformten Gegenstandes gemäß Anspruch 8 oder 9, wobei
eine Temperatur einer Form während der Formgebung 25 bis 55°C beträgt.

11. Ein aus Polyesterharz geformter Gegenstand, hergestellt durch das Verfahren zur Herstellung eines aus Polyesterharz geformten Gegenstandes gemäß einem der Ansprüche 8 bis 10.

## Revendications

1. Méthode de production d'une composition de résine de polyester, la méthode comprenant :
une étape (I-a) d'obtention d'une composition de poly(acide lactique) (X) contenant un poly(acide lactique) (A), du pentaérythritol (C), et un silicate (D) ; et
une étape (II-a) de mélange de la composition de poly(acide lactique) (X) avec un poly(3-hydroxyalcanoate) (B).

2. Méthode de production d'une composition de résine de polyester, la méthode comprenant :
une étape (I-b) d'obtention d'une composition de poly(3-hydroxyalcanoate) (Y) contenant un poly(3-hydroxyalcanoate) (B), du pentaérythritol (C), et un silicate (D) ; et
une étape (II-b) de mélange de la composition de poly(3-hydroxyalcanoate) (Y) avec un poly(acide lactique) (A).

3. Méthode de production d'une composition de résine de polyester selon la revendication 1 ou 2, dans laquelle
le pentaérythritol (C) est mélangé de façon que la quantité du pentaérythritol (C) ne soit pas inférieure à 0,05 partie en poids et pas supérieure à 20 parties en poids pour 100 parties en poids de la quantité totale (A + B) du poly(acide lactique) (A) et du poly(3-hydroxyalcanoate) (B).

4. Méthode de production d'une composition de résine de polyester selon l'une quelconque des revendications 1 à 3, dans laquelle
le silicate (D) est mélangé de façon que la quantité du silicate (D) ne soit pas inférieure à 10 parties en poids et pas supérieure à 40 parties en poids pour 100 parties en poids de la quantité totale (A + B) du poly(acide lactique) (A) et du poly(3-hydroxyalcanoate) (B).

5. Méthode de production d'une composition de résine de polyester selon l'une quelconque des revendications 1 à 4, dans laquelle
le poly(acide lactique) (A) est mélangé de façon que la quantité du poly(acide lactique) (A) ne soit pas inférieure à 55 parties en poids et pas supérieure à 75 parties en poids pour 100 parties en poids de la quantité totale (A + B) du poly(acide lactique) (A) et du poly(3-hydroxyalcanoate) (B).

6. Méthode de production d'une composition de résine de polyester selon l'une quelconque des revendications 1 à 5, dans laquelle
le poly(3-hydroxyalcanoate) (B) est au moins l'un choisi dans le groupe constitué par un poly(3-hydroxybutyrate), un poly(3-hydroxybutyrate-co-3-hydroxyvalérate), un poly(3-hydroxybutyrate-co-3-hydroxyvalérate-co-3-hydroxyhexanoate), un poly(3-hydroxybutyrate-co-3-hydroxyhexanoate), et un poly(3-hydroxybutyrate-co-4-hydroxybutyrate).

7. Composition de résine de polyester fabriquée par la méthode de production d'une composition de résine de polyester selon l'une quelconque des revendications 1 à 6.

8. Méthode de production d'un article formé d'une résine de polyester, la méthode comprenant, après l'étape (II-a) selon la revendication 1, la soumission de la composition de résine de polyester à une mise en forme.

9. Méthode de production d'un article formé d'une résine de polyester, la méthode comprenant, après l'étape (II-b) selon la revendication 2, la soumission de la composition de résine de polyester à une mise en forme.

10. Méthode de production d'un article formé d'une résine de polyester selon la revendication 8 ou 9, dans lequel la température du moule durant la mise en forme est de 25 à 55°C.

11. Article formé d'une résine de polyester produit par la méthode de production d'un article formé d'une résine de polyester selon l'une quelconque des revendications 8 à 10.
